# EUROPÄISCHE PATENTSCHRIFT

(11) **EP 3 122 315 B1**
(45) Veröffentlichungstag und Bekanntmachung des Hinweises auf die Patenterteilung: **14.11.2018**
(21) Anmeldenummer: 15701352.5
(22) Anmeldetag: 27.01.2015
(51) Int. Cl.: A61K 8/35, A61K 8/37, A61Q 17/04, A61Q 19/00, A61Q 19/08

(54) **WIRKSTOFFKOMBINATIONEN AUS 4-HYDROXYACETOPHENON UND EINEM ODER MEHREREN GLYCERIN- UND/ODER OLIGOGLYCERINESTER VON VERZWEIGTKETTIGEN UND/ODER UNVERZWEIGTKETTIGEN ALKANCARBONSÄUREN, SOWIE KOSMETISCHE ODER DERMATOLOGISCHE ZUBEREITUNGEN, DIESE WIRKSTOFFKOMBINATIONEN ENTHALTEND**
ACTIVE-INGREDIENT COMBINATIONS CONSISTING OF 4-HYDROXYACETOPHENONE AND ONE OR MORE GLYCERIN AND/OR OLIGOGLYCERIN ESTERS OF BRANCHED CHAINS AND/OR UNBRANCHED CHAINS OF ALKANE CARBOXYLIC ACIDS, AND COSMETIC OR DERMATOLOGICAL PREPARATIONS CONTAINING SAID ACTIVE-INGREDIENT COMBINATIONS
ASSOCIATIONS DE PRINCIPES ACTIFS CONSTITUÉES DE 4-HYDROXYACÉTOPHÉNONE ET D'UN OU DE PLUSIEURS ESTERS DE GLYCÉROL ET/OU D'OLIGOGLYCÉROL D'ACIDES ALCANE-CARBOXYLIQUES À CHAÎNES RAMIFIÉES ET/OU À CHAÎNES NON RAMIFIÉES, ET PRÉPARATIONS COSMÉTIQUES OU DERMATOLOGIQUES CONTENANT CES ASSOCIATIONS DE PRINCIPES ACTIFS

(30) Priorität: 26.03.2014 DE 102014104258
(43) Veröffentlichungstag der Anmeldung: 01.02.2017
(73) Patentinhaber: Symrise AG, 37603 Holzminden (DE)
(72) Erfinder: PRUNS, Julia, 20144 Hamburg (DE); NISSEN, Bente, 20144 Hamburg (DE); RASCHKE, Thomas, 25421 Pinneberg (DE); VON WEDEL-PARLOW, Magdalena, 20251 Hamburg (DE)
(74) Vertreter: Fabry, Bernd
(86) Internationale Anmeldenummer: PCT/EP2015/051544
(87) Internationale Veröffentlichungsnummer: WO 2015/144326

(56) Entgegenhaltungen:
- EP-A1- 2 774 481
- EP-A1- 2 774 604
- DE-A1-102006 027 138
- JP-A- H07 206 645
- KR-A- 20130 134 976
- US-A1- 2011 112 045
- SYMRISE: "Multiple Benefits for Cosmetics with SymSave H", INTERNET CITATION, 26. Juli 2013 (2013-07-26), XP007923019, Gefunden im Internet: URL:http://www.symrise.com/newsroom/articl e/multiple-benefits-for-cosmetics-with-sym saveR-h/ [gefunden am 2015-02-25]
- RAJABI L ET AL: "Acetophenones with selective antimycobacterial activity", LETTERS IN APPLIED MICROBIOLOGY, OXFORD, GB , Bd. 40, Nr. 3 1. März 2005 (2005-03-01), Seiten 212-217, XP002693043, ISSN: 1472-765X, DOI: 10.1111/J.1472-765X.2005.01657.X Gefunden im Internet: URL:http://onlinelibrary.wiley.com/doi/10. 1111/j.1472-765X.2005.01657.x/full [gefunden am 2013-02-18]

## Beschreibung

### GEBIET DER ERFINDUNG

Die vorliegende Erfindung betrifft Wirkstoffkombinationen aus 4-Hydroxyacetophenon und aus einem oder mehreren Glycerin- und/oder Oligoglycerinestern von verzweigtkettigen und/oder unverzweigtkettigen Alkancarbonsäuren sowie kosmetische oder dermatologische Zubereitungen, diese Wirkstoffkombinationen enthaltend.

Die vorliegende Erfindung betrifft kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

### STAND DER TECHNIK

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen. Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesem Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionsweise der Hornschicht entscheidend beeinträchtigt.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze noch verstärkt werden kann.

Bei gesunder Haut sind diese Vorgänge im Allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muss durch externe Maßnahmen regeneriert werden.

Darüber hinaus ist bekannt, dass Lipidzusammensetzung und -menge der Hornschicht der pathologisch veränderten, trockenen und der trockenen, jedoch nicht erkrankten Haut jüngerer und älterer Menschen vom Normalzustand abweicht, der in der gesunden, normal hydrierten Haut einer gleichalten Altersgruppe vorgefunden wird. Dabei stellen die Veränderungen im Lipidmuster der sehr trockenen, nicht-ekzematösen Haut von Patienten mit atopischem Ekzem einen Extremfall für die Abweichungen dar, die in der trockenen Haut hautgesunder Menschen vorgefunden werden.

Die Wirkung von Salben und Cremes auf Barrierefunktion und Hydratation der Hornschicht besteht in der Regel nicht in einer Wiederherstellung bzw. Stärkung der physikalischchemischen Eigenschaften der Lamellen aus Interzellularlipiden. Ein wesentlicher Teileffekt beruht auf der bloßen Abdeckung der behandelten Hautbezirke und dem daraus resultierenden Wasserstau in der darunterliegenden Hornschicht. Co-applizierte hygroskopische Substanzen binden das Wasser, so dass es zu einer messbaren Zunahme des Wassergehaltes in der Hornschicht kommt. Diese rein physikalische Barriere kann jedoch relativ leicht wieder entfernt werden. Nach dem Absetzen des Produktes kehrt die Haut dann sehr schnell wieder den Zustand vor Behandlungsbeginn zurück. Darüber hinaus kann die Hautpflegewirkung bei regelmäßiger Behandlung nachlassen, so dass schließlich sogar während der Behandlung der Status quo wieder erreicht wird. Bei bestimmten Produkten verschlechtert sich der Zustand der Haut nach Absetzen unter Umständen vorübergehend. Eine nachhaltige Produktwirkung wird in der Regel also nicht oder nur in einem eingeschränkten Masse erreicht.

Um die defizitäre Haut bei ihrer natürlichen Regeneration zu unterstützen und ihre physiologische Funktion zu stärken, werden topischen Präparaten in neuerer Zeit zunehmend Interzellularlipidmischungen zugesetzt, die von der Haut zum Wiederaufbau der natürlichen Barriere verwendet werden sollen. Allerdings handelt es sich bei diesen Lipiden, insbesondere aber den Ceramiden, um sehr teure Rohstoffe. Zudem ist ihre Wirkung meist sehr viel geringer als erhofft. Ziel der vorliegenden Erfindung war es somit, Wege zu finden, die Nachteile des Standes der Technik zu vermeiden. Insbesondere sollte die Wirkung der Hautpflegeprodukte physiologisch, schnell und nachhaltig sein.

Unter Hautpflege im Sinne der vorliegenden Erfindung ist in erster Linie zu verstehen, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, Lipide, Elektrolyte) gestärkt oder wiederhergestellt wird.

Produkte zur Pflege, Behandlung und Reinigung trockener und strapazierter Haut sind an sich bekannt. Allerdings ist ihr Beitrag zur Regeneration einer physiologisch intakten, hydratisierten und glatten Hornschicht umfangmäßig und zeitlich begrenzt.

Die Wirkung von Salben und Cremes auf die Barrierefunktion und die Hydratation der Hornschicht beruht im Wesentlichen auf der Abdeckung (Okklusion) der behandelten Hautbezirke. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen sogar vorübergehend. Eine nachhaltige Produktwirkung wird in der Regel also nicht oder nur in einem eingeschränkten Masse erreicht.

Die Wirkung einiger pharmazeutischer Zubereitungen auf die Barrierefunktion der Haut besteht sogar in einer selektiven Barriereschädigung, die ermöglichen soll, dass Wirkstoffe in bzw. durch die Haut in den Körper eindringen können. Ein gestörtes Erscheinungsbild der Haut wird dabei als Nebenwirkung teilweise billigend in Kauf genommen.

Die Wirkung von pflegenden Reinigungsprodukten besteht im Wesentlichen in einer effizienten Rückleitung mit Sebumlipid-ähnlichen Substanzen. Durch die gleichzeitige Verminderung des Tensidgehalts solcher Zubereitungen lässt sich der Schaden an der Hornschichtbarriere weiter begrenzen.

Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung der Hautalterung, insbesondere der durch oxidative Prozesse hervorgerufenen Hautalterung.

Antioxidantien sind Substanzen, welche Oxidationsprozesse verhindern bzw. welche die Autoxodation ungesättigte Verbindungen enthaltender Fette verhindern. Antioxidantien, welche auch auf dem Gebiete der Kosmetik und Pharmazie Verwendung finden sind beispielsweise alpha-Tocopherol, Sesamol, Gallensäurederivate, Butylhydroxyanisol und Butylhydroxytoluol.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz **"**Skin Diseases Associated with Oxidative Injury" in "Oxidative Stress in Dermatology", S. 323 und folgende (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

UV-Strahlung beispielsweise kann zu photooxidativen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxyradikale, Hydroperoxyradikale sowie Superoxidionen. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nicht-radikalischer angeregter Zustand des Sauerstoffmoleküls, kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Auch aus dem Grunde, solchen Reaktionen vorzubeugen, können kosmetischen bzw. dermatologischen Formulierungen zusätzlich Antioxidantien und/oder Radikalfänger einverleibt werden. Neben der UV-Strahlung induzieren auch andere Umweltnoxen wie z.B. Ozon, Zigarattenrauch, oxidierende Chemikalien, Metallionen (Eisen, Nickel, Kupfer usw.) Schwefel- und Stickoxide oxidativen Stress in der Haut und leisten so vorzeitiger Hautalterung Vorschub.

Auch Hitzeeinwirkung kann zu einem beachtlichen Anstieg des Lipidperoxidspiegels der beeinträchtigten Haut führen, und sogar normale Stoffwechselvorgänge können oxidativen Stress induzieren.

### AUFGABE DER ERFINDUNG

Aufgabe der vorliegenden Erfindung war, den Nachteilen des Standes der Technik Abhilfe zu schaffen. Insbesondere war eine Aufgabe der vorliegenden Erfindung, Wirkstoffe und Produkte zur Verfügung zu stellen, welche in vivo als Antioxidantien wirken und/oder der durch oxidative Beanspruchung hervorgerufenen Hautalterung entgegenzuwirken.

Ein bekanntes und hochwirksames Antioxidans ist das 4-Hydroxyacetophenon, welches unter anderem von der Gesellschaft Symrise unter der Handelsbezeichnung Symsave(R) H" verkauft wird (siehe https://www.symrise.com/newsroom/ article/multiple-benefits-for-cosmetics-with-symsaveR-h/). Es hat die CAS-Nr. 99-93-4 und zeichnet sich durch folgende chemische Struktur aus:

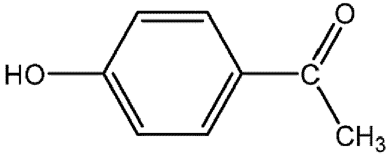

Nachteil dieser Substanz ist, dass ihre Löslichkeit in kosmetischen Lösungsmitteln wie Ölen oder Lipiden nur mäßig ist, was ihren Einsatz in - meistens ölhaltigen - kosmetischen oder dermatologischen Zubereitungen begrenzt.

Aufgabe der vorliegenden Erfindung war es also, diesem Übelstande abzuhelfen und Wege aufzuzeigen, wie die Einsatzkonzentration von 4-Hydroxyacetophenon in gelöster Form erhöht werden kann.

### BESCHREIBUNG DER ERFINDUNG

Gegenstand der Erfindung Wirkstoffkombination enthaltend oder bestehend aus
(a) 4-Hydroxyacetophenon und
(b) mindestens einem Monoester von
   (b1) Glycerin mit einer linearen oder verzweigten Alkancarbonsäure mit 6 bis 14 Kohlenstoffatomen und/oder
   (b2) Oligoglycerin mit einer linearen oder verzweigten Alkancarbonsäure.

Es war überraschend und für den Fachmann nicht vorauszusehen, dass Wirkstoffkombinationen aus einem oder mehreren solcher Glycerin- und/oder Oligoglycerinester von verzweigtkettigen und/oder unverzweigtkettigen Alkancarbonsäuren und 4-Hydroxyacetophenon sowie kosmetische oder dermatologische Zubereitungen, diese Wirkstoffkombinationen enthaltend, den Nachteilen des Standes der Technik abhelfen.

### GLYCERIN -UND OLIGOGLYCERINESTER

Der oder die erfindungsgemäß vorteilhaften Glycerin- und/oder Oligoglycerinester von verzweigtkettigen und/oder unverzweigtkettigen Alkancarbonsäuren sind bevorzugt eine oder mehrere Substanzen, gewählt aus der Gruppe
- der Monoglycerin-monocarbonsäuremonoester,
- der Diglycerin-monocarbonsäuremonoester,
- der Triglycerin-monocarbonsäuremonoester,
- der Monoglycerin-dicarbonsäuremonoester,
- der Diglycerin-dicarbonsäuremonoester und
- der Triglycerin-dicarbonsäuremonoester.

Die erfindungsgemäßen Monoglycerin-mono- bzw. -dicarbonsäuremonoester werden durch die allgemeine Formel 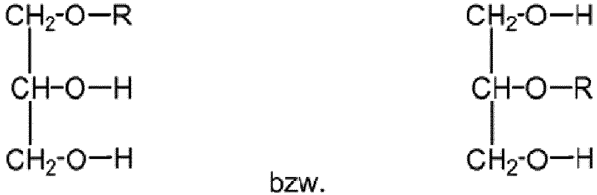 wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die folgenden:

| | | |
|---|---|---|
| Hexansäure | Capronsäure | R = -C(O)-C₅H₁₁) |
| Heptansäure | Önanthsäure | R = -C(O)-C₆H₁₃) |
| Octansäure | Caprylsäure | R = -C(O)-C₇H₁₅) |
| Nonansäure | Pelargonsäure | R = -C(O)-C₈H₁₇) |
| Decansäure | Caprinsäure | R = -C(O)-C₉H₁₉) |
| Undecansäure | | R = -C(O)-C₁₀H₂₁₎ |
| Undecensäure | | R = -C(O)C₁₀H₁₉) |
| Dodecansäure | Laurinsäure | R = -C(O)-C₁₁H₂₃) |
| Tridecansäure | | R = -C(O)-C₁₂H₂₃5 |
| Tetradecansäure | Myristinsäure | R = -C(O)-C₁₃H₂₇), |

Besonders vorteilhaft stellt R den Octanoylrest (Caprylsäurerest) bzw. den Decanoylrest (Caprinsäurerest) dar, wird also durch die Formeln

R = -C(O)-C₇H₁₅ bzw. R = -C(O)-C₉H₁₉

repräsentiert. In dieser Schrift, insbesondere in den Beispielen, werden folgende Kürzel verwendet:
GMCy für Glycerinmonocaprylat,
GMC für Glycerinmonocaprinat,
GML für Glycerinmonolaurat,
GMS für Glycerinmonostearat,
GMU für Glycerinmonoundecylat.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration. hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.- %, bevorzugt 0,1 bis 5 Gew.- %, besonders bevorzugt 0,2 - 2 Gew.-%, jeweils bezogen auf das Gesamtgewicht der jeweiligen Formulierung.

Erfindungsgemäß liegen die Di- bzw. Triglycerineinheiten der erfindungsgemäßen Diglycerin-mono- bzw. -dicarbonsäure-monoester bzw. Triglycerin-mono- bzw. -dicarbonsäure-monoester als lineare, unverzweigte Moleküle, also über die jeweiligen OH-Gruppen in 1- bzw. 3- Stellung veretherte "Monoglycerinmoleküle" vor.

Ein geringer Anteil zyklischer Di- bzw. Triglycerineinheiten sowie über die OH-Gruppen in 2- Stellung veretherte Glycerinmoleküle kann geduldet werden. Es ist jedoch von Vorteil, solche Verunreinigungen so gering wie nur möglich zu halten.

Die erfindungsgemäßen Mono- bzw. Dicarbonsäuremonoester des Diglycerins sind bevorzugt durch folgende Struktur gekennzeichnet (Substitutionspositionen angegeben): 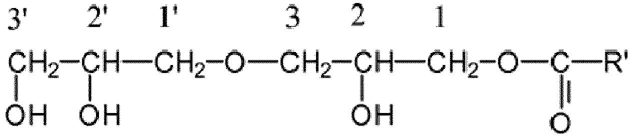 wobei R' einen Kohlenwasserstoffrest, vorteilhaft einen verzweigten oder unverzweigten Alkyl- oder Alkenylrest von 5 bis 17 C-Atomen darstellt. Die erfindungsgemäßen Mono- bzw. Dicarbonsäuremonoester des Triglycerins sind bevorzugt durch folgende Struktur gekennzeichnet (Substitutionspositionen angegeben): 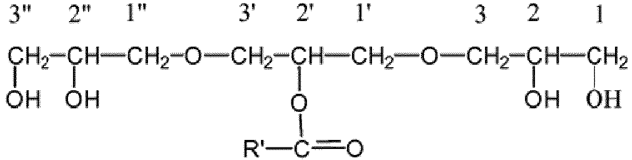 wobei R" einen Kohlenwasserstoffrest, vorteilhaft einen verzweigten oder unverzweigten Alkyl- oder Alkenylrest Alkylrest von 5 bis 17 C-Atomen darstellt. Die diesen Estern zugrundeliegenden Monocarbonsäuren sind die folgenden:

| | | |
|---|---|---|
| Hexansäure | Capronsäure | R' bzw. R" = -C₅H₁₁ |
| Heptansäure | Önanthsäure | R' bzw. R" = -C₆H₁₃ |
| Octansäure | Caprylsäure | R' bzw. R" = -C₇H₁₅ |
| Nonansäure | Pelargonsäure | R' bzw. R" = -C₈H₁₇ |
| Decansäure | Caprinsäure | R' bzw. R" = -C₉H₁₉ |
| Undecansäure | | R' bzw. R" = -C₁₀H₂₁ |
| 10-Undecensäure | Undecylensäure | R' bzw. R" = -C₁₀H₁₉ |
| Dodecansäure | Laurinsäure | R' bzw. R" = -C₁₁H₂₃ |
| Tridecansäure | | R' bzw. R" = -C₁₂H₂₃ |
| Tetradecansäure | Myristinsäure | R' bzw. R" = -C₁₃H₂₇ |
| Pentadecansäure | | R' bzw. R"= -C₁₄H₂₉ |
| Hexadecansäure | Palmitinsäure | R' bzw. R"= -C₁₅H₃₁ |
| Heptadecansäure | Margarinsäure | R' bzw. R"= -C₁₆H₃₃ |
| Octadecansäure | Stearinsäure | R' bzw. R"= -C₁₇H₃₅ |

Besonders günstig werden R' und R" gewählt aus der Gruppe der unverzweigten Alkylreste mit ungeraden C-Zahlen, insbesondere mit 9, 11 und 13 C-Atomen.

Im Allgemeinen sind die Monocarbonsäuremonoester des Diglycerins denen des Triglycerins bevorzugt. Ganz besonders günstig sind:

| | | |
|---|---|---|
| Diglycerinmonocaprylat | DMCy | R' = 7 |
| Diglycerinmonocaprinat | DFMC | R' = 9 |
| Diglycerinmonolaurat | DML | R' = 11 |
| Triglycerinmonolaurat | TML | R" = 11 |
| Diglycerinmonolaurat | DML | R' = 11 |
| Triglycerinmonomyristat | TMM | R" = 13 |

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1- Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2"-Position Asymmetriezentren. Erfindungsgemäß aktiv und gleichermaßen von Vorteil sind die 2S2'S2"S-, die 2R2'S2"S-, die 2S2'R2"S-, die 2R2'R2"S-, die 2S2'S2"R, die 2R2'S2"R-, die 2S2'R2"R- und die 2R2'R2"R- Konfiguration.

Es hat sich als günstig herausgestellt, racemische Gemische der Stereoisomere zu verwenden.

Es ist von Vorteil, den Gehalt an (i) Mono-, Di- und/oder Triglycerin-mono- bzw. dicarbonsäuremonoester und (ii) Dialkylcarbonsäure bzw. -säuren so zu wählen, dass Verhältnisse (i): (ii) wie 10: 1 bis 1: 10, insbesondere wie etwa 5: 1 bis 1: 5, ganz besonders vorteilhaft wie etwa 2: 1 bis 1: 2: 5 entstehen.

Ein besonders bevorzugter Glycerinfettsäureester ist das Glycerylcaprylat. Handelsübliche Rohstoffe enthalten üblicherweise Glycerinester mit einer Mischung von Fettsäuren unterschiedlicher Kettenlänge. Erfindungsgemäße Glycerylcaprylate können somit Mischungen von Fettsäuren mit einer Kettenlänge von überwiegend C6 bis C12 enthalten. Bevorzugte Glycerylcaprylate, welche aus Mischungen von mittelkettigen Fettsäuren aufgebaut sind, enthalten typischerweise eine mittlere Fettsäurekettenlänge von circa. C8. Ein bevorzugter Vertreter dieser Glycerylcaprylate hat die CAS Nummer 26402-26-6.

Ein weiterer besonders bevorzugter Glycerinfettsäureester ist das Polyglyceryl-2-caprat beziehungsweise Polygylceryl-2-monodecanoat. Handelsübliche Substanzen dieser Glycerinfettsäureester stellen Gemische von im Mittel Diglycerinen mit Fettsäuren dar, die eine mittlere Kettenlänge von circa C10 aufweisen. Hierbei treten typischerweise konkrete Fettsäurekettenlängen von überwiegend C6 bis C14 auf. Ein bevorzugter Vertreter dieser Substanzklasse hat die CAS Nummer 156153-06-9.

Erfindungsgemäße Zubereitungen, die erfindungsgemäßen Wirkstoffkombinationen enthaltend, sind besonders vorteilhaft dadurch gekennzeichnet, dass die erfindungsgemäßen Wirkstoffkombinationen in Konzentrationen von 0,05 bis 10,00 Gew.- %, bevorzugt 0,1 bis 5,0 Gew.- %, vorliegen, jeweils bezogen auf das Gesamtgewicht der Zubereitungen.

Vorteilhaft werden Gewichtsverhältnisse von der Gesamtmenge an aus einem oder mehreren Glycerin- und/oder Oligoglycerinester von verzweigtkettigen und/oder unverzweigtkettigen Alkancarbonsäuren zu der Menge an 4-Hydroxyacetophenon aus dem Bereich von 20 zu 1 bis 1 zu 20 gewählt werden, bevorzugt von 10 zu 1 bis 1 zu 10, insbesondere bevorzugt von 5 zu 1 bis 1 zu 5.

Bevorzugte Einsatzkonzentrationen von 4-Hydroxyacetophenon in kosmetischen oder dermatologischen Zubereitungen werden aus dem Bereich von 0,001 % bis 2 % gewählt, bevorzugt von 0,01 % bis 1,5 %, insbesondere bevorzugt von 0,1 % bis 1 %, jeweils bezogen auf das Gesamtgewicht der Zubereitungen.

### ANTIOXIDANTIEN

Erfindungsgemäße Zubereitungen können einen zusätzlichen Gehalt an Antioxidantien enthalten.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäu- re) und deren Derivate, Peptide wie D,L-Carnosin, D-Carnosin, L-Carnosin und deren Derivate (z.B. Anserin), Carotinoide, Carotine (z.B. a-Carotin, beta-Carotin, Lycopin) und deren Derivate, Chlorogensäure und deren Derivate, Liponsäure und deren Derivate Thioredoxin, Glutathion, Cystein, Cystin, Cystamin und deren Glycosyl-, N-Acetyl-, Methyl-, Ethyl-, Pro- pyl-, Amyl-, Butyl- und Lauryl-, Palmitoyl-, Oleyl-, gamma-Linoleyl-,Cholesteryl- und Glycerylester sowie deren Salze, Dilaurylthiodipropionat, ferner (Metall)-Chelatoren (z.B. a-Hydroxyfettsäuren, Palmitinsäure, Phytinsäure), a-Hydroxysäuren (z.B. Citronensäure, Milchsäure, Apfelsäure), EDTA, EGTA und deren Derivate, ungesättigte Fettsäuren und deren Derivate (z.B. gamma-Linolensäure, Linolsäure, ölsäure), Folsäure und deren Derivate, Ubichinon und Ubichinol und deren Derivate, Vitamin C und Derivate (z.B. Ascorbylpalmitat, Mg- Ascorbylphosphat, Ascorbylacetat), Tocopherole und Derivate (z.B. Vitamin-E-acetat), Vitamin A und Derivate (Vitamin-A-palmitat) sowie Rutinsäure und deren Derivate, a- Glycosylrutin, Ferulasäure, Furfurylidenglucitol, Butylhydroxytoluol, Harnsäure und deren Derivate, Harnstoff, Panthenol, Zink und dessen Derivate (z.B. ZnO),Ti02, Resveratrol, Mag- nolienrindenextrakt (enthaltend Magnolol, Honokiol) und die erfindungsgemäss geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 10 Gew.- %, besonders bevorzugt 0,05 bis 5 Gew.- %, insbesondere 0,1 bis 2 Gew.- %, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 5 Gew.- %, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 1 Gew.- %, bezogen auf das Gesamtgewicht der Formulierung, zu wählen.

### FORMULIERUNGEN

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen. Es ist auch gegebenenfalls vorteilhaft, die Zusammensetzungen gemäß der Erfindung abzupuffern. Vorteilhaft ist ein pH-Bereich von 3,5 - 8,0. Besonders günstig ist es, den pH-Wert in einem Bereich von 4,5 - 7,5 zu wählen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. Üblicherweise für diesen Typ von Zubereitungen eingesetzt werden. So können sie z.B. eine Lösung, eine Emulsion vom Typ Wasser-in-ÖI (W/O) oder vom Typ öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate. Emulsionen sind gemäß der Erfindung vorteilhaft und enthalten z.B. die genannten Fette, öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes öl in Gegenwart eines Verdickungsmittels, das bei ölig-alkoholischen Gelen vorzugsweise Siliziumdioxid oder ein Aluminiumsilikat, bei wässrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.- % bis 30 Gew.- %, vorzugsweise 0,5 bis 10 Gew.- %, insbesondere 1 bis 6 Gew.- % beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Föhnen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nichtionische oder amphotere oberflächenaktive Substanz oder Gemische daraus, mindestens eine dialkylsubstituierte Carbonsäure im wässrigen Medium und Hilfsmittel, wie sie üblicherweise dafür verwendet werden.

### GEWERBLICHE ANWENDBARKEIT

Ein weiterer Gegenstand der Erfindung betrifft die Verwendung von Monoestern von Glycerin mit einer linearen oder verzweigten Alkancarbonsäure mit 6 bis 14 Kohlenstoffatomen und/oder Oligoglycerin mit einer linearen oder verzweigten Alkancarbonsäure zur Verbesserung der Löslichkeit von 4-Hydroxyacetophenon in ölhaltigen Zubereitungen oder kosmetischen Ölen.

Die folgenden Beispiele sollen die Verkörperungen der vorliegenden Erfindungen verdeutlichen. Die Angaben beziehen sich stets auf Gew.-%, sofern nicht andere Angaben gemacht werden.

### LÖSLICHKEITSVERSUCHE

Die Durchführung der Löslichkeitsversuche erfolgte bei 2 verschiedenen Temperaturen: Bei Raumtemperatur (ca. 20 °C) und bei 35 °C.

Das weiße, kristalline 4-Hydroxyacetophenon ("4-HAP") wurde in kleinen Portionen langsam (über mehrere Stunden) zum jeweiligen Lösungsmittel unter Rühren hinzugegeben, bis sich kein weiteres Material mehr lösen ließ. Hier war die Löslichkeitsgrenze erreicht. Nicht gelöstes Material war deutlich als milchiger Niederschlag oder einzelne Kristalle erkennbar. Das Additiv (der jeweilige Glycerinester) wurde ebenfalls in kleinen Portionen hinzugegeben.

1 % des jeweiligen Glycerylesters (Glycerylcaprylat, Polyglyceryl-2-caprat) lösten sich im verwendeten Lösungsmittel (kosmetischen öl) vollständig bei RT und 35°C.

Die Experimente bei 35°C wurden im Wasserbad durchgeführt. Die Temperatur wurde per Temperatursonde, welche mit dem Heizrührer verbunden war, konstant gehalten. Die Experimente wurden in verschraubten 15 ml Gläsern durchgeführt, um ein Verdampfen des Lösungsmittels zu verhindern. Die Löslichkeit wurde 12-24 Stunden nach der letzten Substanzzugabe gemessen, so dass sich das System equilibrieren konnte. Bekannte Emulgatoren wie Glycerylstearate und Glycerylstearatcitrate zeigten keinen Einfluss auf die Löslichkeit von 4-Hydroxyacetophenon. Das ist überraschend, da Emulgatoren als grenzflächenaktive Substanzen bekanntermaßen einen positiven Einfluss auf die Löslichkeit von kosmetischen Rohstoffen haben. Die Löslichkeitsversuche wurden in dem bekannten kosmetischen Öl "C12-15 Alkylbenzoate", erhältlich unter anderem unter dem Handelsnamen Tegesoft® TN B von der Firma Evonik, durchgeführt. Die Ergebnisse sind in **Tabelle 1** wiedergegeben:

**Tabelle 1**

| Löslichkeitsversuche | | |
|---|---|---|
| **Glycerinester** | **Löslichkeit "HAP" bei RT** | **Löslichkeit "HAP" bei 35 °C** |
| Ohne | 2 % gelöst (Kristalle bei 2,25 %) | 2,5 % gelöst (Kristalle mit 3 %) |
| 1 % Glycerylcaprylat | 2,25 % gelöst | 3 % gelöst (Steigerung im Vergleich zu 2,5 % ohne Ester |
| 1 % Polyglyceryl-2-caprat | 2,25 % nicht vollständig gelöst | 3 % gelöst (Steigerung im Vergleich zu 2,5 % ohne Ester) |
| 0,5 % Polyglyceryl-2-caprat | 2,25 % gelöst | |

### ANWENDUNGSBEISPIELE

| **Beispielrezeptur 1 : O/W Tagescreme** | **Gew.-%** |
|---|---|
| PEG-40 Stearat | 1,5 |
| Glycerylstearat | 1 |
| Cetylalkohol | 3 |
| Dimethicon | 2 |
| C12-15 Alkylbenzoat | 5 |
| Dicaprylylether | 2 |
| Octyl Methoxycinnamat | 5 |
| Butylmethoxydibenzoylmethan (z.B. Parsol® 1789) | 1,5 |
| 4-Hydroxyacetophenon | 0,5 |
| AcrylaUC10-30 Alkyl Acrylat Crosspolymer | 0,2 |
| Parfüm | 0,2 |
| EDTA | 0,2 |
| Tocopherylacetat | 0,3 |
| Glycerin | 9 |
| Phenoxyethanol | 0,5 |
| Glycerylcaprylat | 0,3 |
| Natronlauge (pH 6 eingestellt) | q.s. |
| Wasser | ad 100 |

| **Beispielrezeptur 2: O/W Nachtcreme** | **Gew.-%** |
|---|---|
| Glycerylstearatcitrat | 2 |
| Glycerylstearat | 2 |
| Stearylalkohol | 2 |
| Cyclomethicon | 3 |
| Dicaprylylether | 3 |
| C12-15 Alkylbenzoat | 5 |
| Neutralfett | 1 |
| Carbomer | 0,3 |
| Methylparaben | 0,3 |
| Polyglyceryl-2-caprat | 0,4 |
| 4-Hydroxyacetophenon | 0,7 |
| Glycerin | 5 |
| EDTA | 0,2 |
| Parfüm | 0,2 |
| Natronlauge (pH 5 eingestellt) | q.s. |
| Wasser | ad 100 |

| **Beispielrezeptur 3: Sonnenschutzcreme** | **Gew.-%** |
|---|---|
| Polyglyceryl-10-Stea rat | 1,5 |
| Glycerylstearat | 3 |
| Octyldodecanol | 3 |
| Butylmethoxydibenzoylmethan (z.B. Parsol® 1789) | 2 |
| Octyl Methoxycinnamat | 5 |
| Titandioxid | 1 |
| Diethylamino Hydroxybenzoyl Hexyl Benzoate | 1 |
| Diethylhexyl Butamido Triazon | 1 |
| C12-15 Alkylbenzoat | 5 |
| Phenoxyethanol | 0,4 |
| Polyacrylsäure | 0,3 |
| Xanthan Gum | 0,1 |
| Butylenglycol | 3 |
| Glycerin | 10 |
| EDTA | 0,1 |
| Parfum | 0,4 |
| Panthenol | 0,5 |
| Glycerylcaprylat | 0,5 |
| 4-Hydroxyacetophenon | 0,2 |
| Natronlauge (pH 6 eingestellt) | q.s. |
| Wasser | ad 100 |

| **Beispielrezeptur 4: O/W Creme** | **Gew.-%** |
|---|---|
| Polyglyceryl-3 Methylglucosedistearat | 3 |
| Glycerylstearat | 1 |
| Behenylalkohol | 1 |
| C12-15 Alkylbenzoat | 6 |
| Dicaprylylcarbonat | 2 |
| Caprylsäure/Caprinsäuretriglycerid | 2 |
| 2,4-Bis-{[4-(2-ethyl-hexyloxy)-2-hydroxy]-phenyl}-6-(4-methoxyphenyl)-1,3,5-triazin (Tinosorb® S) | 1 |
| Octocrylen | 6 |
| Terephthalyliden Dicamphor Sulfonsäure | 1 |
| 4-Hydroxyacetophenon | 1 |
| Polyglyceryl-2-caprat | 0,5 |
| Acryl C10-30 Alkyl Acrylat Crosspolymer | 0,3 |
| Carrageen | 0,1 |
| Glycerin | 6 |
| EDTA | 0,2 |
| Phenoxyethanol | 0,5 |
| Niacinamid | 0,2 |
| Natronlauge (pH 7 eingestellt) | q.s. |
| Wasser | ad 100 |

| **Beispielrezeptur 5: Makroemulsion-Roll-on** | **Gew.-%** |
|---|---|
| Polyethylenglykol(21)stearylether | 3,0 |
| Polyethylenglykol(2)stearylether | 2,0 |
| Polypropylenglykol(15)stearylether | 2,0 |
| EDTA | 0,2 |
| 4-Hydroxyacetophenon | 0,3 |
| Parfum, Antioxidantien | q.s. |
| 2-Ethylhexylglycerinether (Octoxyglycerin) | 0,5 |
| Hexandiol | 2,0 |
| Polyglyceryl-2-caprat | 0,3 |
| Aluminiumchlorohydrat | 5,0 |
| Wasser | ad 100 |

| **Beispielrezeptur 6: Transluzente Mikroemulsion für Zerstäuber** | **Gew.-%** |
|---|---|
| Polyoxyethyl en(20)cetylstearylether | 2,0 |
| Polyoxyethylen(12)cetylstearylether | 1,0 |
| Glycerinstearat | 2,5 |
| Cetylstearylalkohol | 0,5 |
| Cetylpalmitat | 0,5 |
| 4-Hydroxyacetophenon | 0,2 |
| Di-n-Octylether | 8,0 |
| Glycerin | 3,0 |
| Glycerylcaprylat | 0,3 |
| Pentandiol | 2,0 |
| Aluminiumchlorohydrat | 4,0 |
| Parfum, Antioxidantien | q.s. |
| Wasser | ad 100 |

| **Beispielrezeptur 7** | **Gew.-%** |
|---|---|
| Cetylstearylalkohol | 3 |
| Caprylsäure/Caprinsäuretriglycerid | 4 |
| Dicaprylylether | 2 |
| C12-15 Alkylbenzoat | 4 |
| Kalium Cetylphosphat | 0,3 |
| Distärkephosphat | 1 |
| Ammonium AcryloyldimethyltauraUVP Copolymer (Aristoflex AVC®) | 0,5 |
| Xanthan Gum | 0,2 |
| 4-Hydroxyacetophenon | 0,4 |
| Glycerylcaprylat | 0,3 |
| Rucinol | 0,1 |
| Glycerin | 7 |
| EDTA | 0,2 |
| Konservierung | q.s. |
| Natriumbisulfit | 0,1 |
| Natronlauge (pH 5,5 eingestellt) | q.s. |
| Wasser 100% | ad 100 |

## Patentansprüche

1. Wirkstoffkombination enthaltend oder bestehend aus
(a) 4-Hydroxyacetophenon und
(b) mindestens einem Monoester von
(b1) Glycerin mit einer linearen oder verzweigten Alkancarbonsäure mit 6 bis 14 Kohlenstoffatomen und/oder
(b2) Oligoglycerin mit einer linearen oder verzweigten Alkancarbonsäure.

2. Wirkstoffkombination nach Anspruch 1, **dadurch gekennzeichnet, dass** die Komponente (b) Glycerylcaprylat darstellt.

3. Wirkstoffkombination nach Anspruch 1, **dadurch gekennzeichnet, dass** die Komponente (b) Polyglyceryl-2-caprylat darstellt.

4. Wirkstoffkombination nach mindestens einem der Ansprüche 1 bis 3, **dadurch gekennzeichnet, dass** sie die Komponenten (a) und (b) im Gewichtsverhältnis von 1: 20 bis 20:1 enthalten.

5. Kosmetische oder dermatologische Zubereitungen, enthaltend eine Wirkstoffkombination nach einem der vorstehenden Ansprüche 1 bis 4.

6. Zubereitungen nach Anspruch 5, **dadurch gekennzeichnet, dass** sie die Wirkstoffkombination in Mengen von 0,05 bis 10 Gew.-% - bezogen auf die Zubereitungen, enthält.

7. Verwendung von Monoestern von Glycerin mit einer linearen oder verzweigten Alkancarbonsäure mit 6 bis 14 Kohlenstoffatomen und/oder Oligoglycerin mit einer linearen oder verzweigten Alkancarbonsäure zur Verbesserung der Löslichkeit von 4-Hydroxyacetophenon in ölhaltigen Zubereitungen oder kosmetischen Ölen.

## Claims

1. Mixture of active agents comprising or consisting of:
(a) 4-hydroxyacetophenone and
(b) at least one monoester of
(b1) glycerol and a linear or branched alkane carboxylic acid having 6 to 14 carbon atoms and/or
(b2) oligoglycerol with a linear or branched alkane carboxylic acid.

2. Mixture of active agents according to Claim 1, **characterized in that** said component (b) represents glyceryl caprylate.

3. Mixture of active agents according to Claim 1, **characterized in that** said component (b) represents polyglyceryl-2-caprylate.

4. Mixture of active agents according to any of Claims 1 to 3, **characterized in that** they comprise said components (a) and (b) in a ratio by weight of from 1:20 to 20:1.

5. Cosmetic or dermatologic compositions comprising a mixture of active agents according to any of the preceding Claims 1 to 4.

6. Composition according to Claim 5, **characterized in that** they comprise said mixture in amounts of from 0.05 to 10 % by weight - calculated on said composition.

7. Use of monoesters of glycerol with linear or branched alkane carboxylic acids having 6 to 14 carbon atoms and/or oligoglycerol with linear or branched alkane carboxylic acids for improving solubility of 4-hydroxyacetophenone in oil-containing compositions or cosmetic oils.

## Revendications

1. Combinaison d'agents actifs contenant ou constituée par :
(a) de la 4-hydroxyacétophénone et
(b) au moins un monoester de
(b1) glycérine avec un acide alcanecarboxylique linéaire ou ramifié de 6 à 14 atomes de carbone et/ou
(b2) oligoglycérine avec un acide alcanecarboxylique linéaire ou ramifié.

2. Combinaison d'agents actifs selon la revendication 1, **caractérisée en ce que** le composant (b) consiste en du caprylate de glycéryle.

3. Combinaison d'agents actifs selon la revendication 1, **caractérisée en ce que** le composant (b) consiste en du 2-caprylate de polyglycéryle.

4. Combinaison d'agents actifs selon au moins l'une quelconque des revendications 1 à 3, **caractérisée en ce qu'**elle contient les composants (a) et (b) en un rapport en poids de 1:20 à 20:1.

5. Préparations cosmétiques ou dermatologiques, contenant une combinaison d'agents actifs selon l'une quelconque des revendications 1 à 4 précédentes.

6. Préparations selon la revendication 5, **caractérisées en ce qu'**elles contiennent la combinaison d'agents actifs en quantités de 0,05 à 10 % en poids, par rapport aux préparations.

7. Utilisation de monoesters de glycérine avec un acide alcanecarboxylique linéaire ou ramifié de 6 à 14 atomes de carbone et/ou d'oligoglycérine avec un acide alcanecarboxylique linéaire ou ramifié pour améliorer la solubilité de 4-hydroxyacétophénone dans des préparations contenant des huiles ou des huiles cosmétiques.
